# EUROPÄISCHE PATENTANMELDUNG

(11) **EP 4 675 552 A1**
(43) Veröffentlichungstag der Anmeldung: **07.01.2026**
(21) Anmeldenummer: 24186479.2
(22) Anmeldetag: 04.07.2024
(51) Int. Cl.: G06T 7/00, G06T 7/33, G06V 10/74, G06V 10/94, G06V 20/52, G01N 21/898, G01N 33/46

(54) **VERFAHREN ZUR ERFASSUNG VON HOLZDEFEKTEN SOWIE HOLZDEFEKTEIGENSCHAFTEN BEI DER VERARBEITUNG VON BAUMSTÄMMEN UND VORRICHTUNG ZUR VERARBEITUNG VON BAUMSTÄMMEN**

(71) Anmelder: Gebrüder Linck, Maschinenfabrik "Gatterlinck" GmbH & Co. KG, 77704 Oberkirch (DE)
(72) Erfinder: Rathke, Jörn, 77704 Oberkirch (DE); Burger, Raphael, 77654 Offenburg (DE)
(74) Vertreter: LBP Lemcke, Brommer & Partner Patentanwälte mbB

(57) **Zusammenfassung**

Verfahren zur Erfassung von Holzdefekten und/oder Holzdefekteigenschaften bei der Verarbeitung eines Baumstamms mit den folgenden Verfahrensschritten:
A) Profilmessung des Baumstammes (1) und Ermittlung einer Stammgeometrie des Baumstamms;
B) Trennende Bearbeitung des Baumstammes (1) unter Erzeugung einer Bearbeitungsoberfläche (8) entlang einer Stammachse des Baumstamms (1);
C) Bildgebende Prüfung des trennend bearbeiteten Baumstammes (1) zumindest an der Bearbeitungsoberfläche (8) und Ermittlung mindestens eines Prüfbildes (14);
D) Ortsaufgelöste Bestimmung eines Holzdefektes (10) und/oder einer Holzdefekteigenschaft an dem trennend bearbeiteten Baumstamm in Abhängigkeit des Prüfbildes (14) aus Verfahrensschritt C) und der Stammgeometrie aus Verfahrensschritt A).

## Beschreibung

Die Erfindung betrifft ein Verfahren zur Erfassung von Holzdefekten und/oder Holzdefekteigenschaften bei der Verarbeitung von Baumstämmen. Ferner betrifft die Erfindung eine Vorrichtung zur Verarbeitung von Baumstämmen.

In der industriellen Holzverarbeitung werden Baumstämme unter anderem zu Brettern verarbeitet. Ein Ziel besteht hierbei darin, eine möglichst hohe Holzausbeute zu erzielen. Dafür wird typischerweise ermittelt, in welcher Anordnung und mit welchen Abmessungen die zu gewinnenden Bretter in Bezug auf die Stammgeometrie angeordnet sein müssen, um die gewünscht hohe Holzausbeute erzielen zu können. Zunächst wird der Baumstamm hierfür mittels eines Profilsensors gescannt. Aus den dabei gewonnenen Messdaten wird eine dreidimensionale Stammgeometrie ermittelt, anhand derer die Anordnung und die Abmessung der zu gewinnenden Bretter bestimmt werden. Dabei befinden sich sogenannte Hauptwarebretter typischerweise in einem innenliegenden Querschnittsbereich eines Baumstammes, während sogenannte Seitenwarebretter in einem demgegenüber außenliegenden Querschnittsbereich angeordnet sind.

Zur Gewinnung eines Seitenwarebretts wird dieses meist unmittelbar am Baumstamm profiliert, wobei der Baumstamm üblicherweise zunächst als sogenanntes Rundholz mit einem im Wesentlichen runden Querschnitts vorliegt, der durch den natürlichen Wuchs des Baumstammes bedingt ist. Für die Profilierung des Seitenwarebretts wird zunächst mindestens ein Schwartenbereich des Baumstammes entfernt und dadurch zumindest eine Bearbeitungsoberfläche erzeugt. In einem derart trennend bearbeiteten Zustand kann der Baumstamm auch nicht nur eine, sondern gleich mehrere umfangseitig verteilte Bearbeitungsoberflächen aufweisen und dabei zum Beispiel als sogenanntes Model mit zwei seitlichen Bearbeitungsoberflächen oder als Kantling mit vier umfangsseitig verteilten Bearbeitungsoberflächen vorliegen.

Nach der trennenden Bearbeitung des Baumstamms wird dieser üblicherweise erneut mittels eines Profilsensors gescannt, um seine Geometrie zu ermitteln. Die dabei erhobenen Geometrieinformationen zum trennend bearbeiteten Baumstamm werden genutzt, um Holzdefekte, wie beispielsweise Risse oder Äste, die sich durch das Stamminnere erstrecken, ortsaufgelöst erkennen zu können. Eine Profilmessung nach der trennenden Bearbeitung ermöglicht es auch, festzustellen, ob die Bearbeitungsoberfläche an dem Baumstamm an der gewünschten Position und Orientierung erzeugt wurde. Dadurch ist es nach der trennenden Bearbeitung möglich, die Abmessungen und Anordnungen der zu gewinnenden Haupt- und Seitenwarebretter gegenüber dem trennend bearbeiteten Baumstamm zu bestimmen oder gegenüber einer ursprünglichen Schnittlösung zu verändern, sodass dieser anschließend entsprechend bearbeitet werden kann.

Es ist ein Nachteil, dass die Profilmessung des trennend bearbeiteten Baumstammes mit einem hohen messtechnischen Aufwand einhergeht. Es ist deshalb die Aufgabe der Erfindung, eine zuverlässige Qualitätsprüfung und eine hohe Holzausbeute bei der Bearbeitung von Baumstämmen bei gleichzeitig geringem messtechnischem Aufwand zu ermöglichen.

Die Aufgabe wird gelöst mittels eines Verfahrens nach Anspruch 1. Vorteilhafte Weiterbildungen sind Gegenstände abhängiger Unteransprüche. Die Aufgabe wird auch gelöst mittels einer Vorrichtung nach Anspruch 12.

Das erfindungsgemäße Verfahren dient der Erfassung von Holzdefekten und/oder Holzdefekteigenschaften bei der Verarbeitung eines Baumstamms und umfasst folgende Verfahrensschritte:
A) Profilmessung des Baumstammes und Ermittlung einer Stammgeometrie des Baumstamms;
B) Trennende Bearbeitung des Baumstammes unter Erzeugung mindestens einer Bearbeitungsoberfläche entlang einer Stammachse des Baumstamms;
C) Bildgebende Prüfung zumindest an der Bearbeitungsoberfläche des trennend bearbeiteten Baumstammes und Erfassung zumindest eines Prüfbildes;
D) Ortsaufgelöste Bestimmung eines Holzdefektes und/oder einer Holzdefekteigenschaft an dem trennend bearbeiteten Baumstamm in Abhängigkeit des Prüfbildes aus Verfahrensschritt C) und der Stammgeometrie aus Verfahrensschritt A).

Der Erfindung liegt die Erkenntnis zugrunde, dass es nicht erforderlich ist, sowohl vor als auch nach der trennenden Bearbeitung des Baumstammes Informationen zu seiner Geometrie zu erheben. Vielmehr beruht die erfinderische Idee darauf, dass die qualitätsrelevanten Informationen, wie etwa eine Position oder räumliche Ausbreitung eines Holzdefektes einerseits anhand der Stammgeometrie des Baumstammes vor seiner trennenden Bearbeitung sowie andererseits anhand von vergleichsweise einfach zu gewinnenden Bilddaten am trennend bearbeiteten Baumstamms gewonnen werden können. Damit ist es entgegen gängiger Praxis auch ohne eine Profilvermessung des trennend bearbeiteten Baumstammes möglich, zu ermitteln, in welcher Anordnung und mit welchen Abmessungen die zu gewinnenden Bretter gegenüber dem trennend bearbeiteten Baumstamm angeordnet sein sollten, um eine möglichst hohe Holzausbeute und eine hohe Schnittholzqualität zu erzielen.

In einer denkbaren Ausführungsform wird der Baumstamm zur Durchführung mindestens eines der Verfahrensschritte A), B), C), D) im Wesentlichen entlang einer Stammachse in einer Vorschubrichtung bewegt. Es ist denkbar, dass die Stammachse gerade verläuft oder mindestens eine oder mehrere Krümmungen aufweist, welche durch den natürlichen Wuchs des Baumstammes bedingt sein können. Dementsprechend kann die Bearbeitungsoberfläche des Baumstammes entlang der Stammachse einen im Wesentlichen geraden oder gekrümmten Verlauf aufweisen.

Vorzugsweise liegt der Baumstamm während Verfahrensschritt A) als Rundholz vor und weist außenumfangsseitig und entlang seiner Stammachse einen im Wesentlichen runden Querschnittsverlauf auf, der durch den natürlichen Wuchs des Baumstammes bedingt ist. Bevorzugt wird Verfahrensschritt A) mittels eines oder mehrerer Profilsensoren durchgeführt, welche jeweils beispielsweise nach Art eines Laserlichtschnittsensors oder eines vergleichbaren Messmittels ausgestaltet sein können und dazu dienen, ein geometrisches Profil des Baumstammes zu ermitteln. Hierbei liegt es im Rahmen der Erfindung, dass der Profilsensor und der Baumstamm sich in einer Relativbewegung zueinander befinden, wobei der Profilsensor insbesondere ruhend gelagert sein kann und der Baumstamm bei einer Vorschubbewegung in Vorschubrichtung gefördert wird. Die Stammgeometrie kann hierbei in Abhängigkeit der erhobenen Profilinformationen und der Vorschubrichtung des Baumstammes ermittelt werden.

Die Stammgeometrie kann im Rahmen der Erfindung durch eine Punktewolke oder vergleichbare Primärdaten repräsentiert sein, welche in Verfahrensschritt A) erhoben werden. Diese Punktewolke kann im Rahmen der Erfindung die Oberfläche des Baumstammes repräsentieren und damit eine räumliche Erstreckung des Baumstammes oder eines Teils des Baumstammes beschreiben. Es liegt auch im Rahmen der Erfindung, dass anhand der Primärdaten ein Stammmodell erzeugt wird, welches die Stammgeometrie repräsentiert. Im Vergleich zu den Primärdaten kann das Stammmodell eine höhere Datendichte aufweisen und hierbei insbesondere eine zumindest bereichsweise geschlossene Oberfläche aufweisen, welche im Wesentlichen der Oberfläche des Baumstammes in Verfahrensschritt A) entspricht. Es ist denkbar, dass das Stammmodell hierbei nach Art eines Hüllmodells vorliegt, welches ausschließlich die äußere Oberfläche des Baumstammes repräsentiert. Ebenso ist denkbar, dass das Stammmodell nach Art eines Massivmodells mit einem virtuellen Stamminneren vorliegt.

Die Erfindung ist nicht darauf beschränkt, auf welche Weise Verfahrensschritt B) durchgeführt wird. Es ist denkbar, dass die trennende Bearbeitung des Baumstammes und die Erzeugung der Bearbeitungsoberfläche mittels eines oder mehrerer Sägemittel erfolgt, wobei ein Schwartenbereich des Baumstammes in einem zusammenhängenden Stück von dem Baumstamm getrennt wird. Ebenso ist denkbar, dass der Schwartenbereich beispielsweise mittels eines Messerkopfes spanend zu Hackschnitzeln verarbeitet wird und dabei die Bearbeitungsoberfäche erzeugt wird.

Die bildgebende Prüfung des trennend bearbeitenden Baumstammes gemäß Verfahrensschritt C) kann mittels eines oder mehrerer bildgebender Sensoren erfolgen, wobei insbesondere auf Kameras oder ein Kamerasystem zurückgegriffen werden kann. Der bildgebende Sensor kann zumindest die Bearbeitungsoberfläche des Baumstammes in dem Prüfbild durch zweidimensional verteilte Graustufen- und/oder RGB-Farbwerte repräsentieren.

Insbesondere kann der mindestens eine bildgebende Sensor derart angeordnet sein, dass der trennend bearbeitete Baumstamm bei der Vorschubbewegung mit seiner Bearbeitungsoberfläche einen Erfassungsbereich des bildgebenden Sensors passiert. Dabei kann der bildgebende Sensor eine Messachse aufweisen, welche eine räumliche Erstreckung seines Erfassungsbereiches angibt und entlang derer der mindestens eine bildgebende Sensor einen Tiefenschärfebereich aufweist. In dem Tiefenschärfebereich ist es mittels des bildgebenden Sensors möglich, hinreichend scharfe Prüfbilder von der Bearbeitungsoberfläche zu erzeugen.

Es ist vorteilhaft, wenn der Sensor während Verfahrensschritt C) mit seiner Messachse im Wesentlichen orthogonal zu der Bearbeitungsoberfläche ausgerichtet ist. Dies kann auf einfache Weise erreicht werden, da die Vorschubbewegung des trennend bearbeiteten Baumstammes typischerweise über eine Mehrzahl von Förderwalzen erzeugt wird, die an ihren Mantelflächen mit der Bearbeitungsoberfläche im Wesentlichen bündig in Kontakt sind. Daher kann der bildgebende Sensor mit seiner Messachse zum Beispiel orthogonal zu den Walzenachsen ausgerichtet werden, um mit seiner Messachse senkrecht zu der Bearbeitungsoberfläche eines trennend bearbeiteten Baumstamms ausgerichtet werden zu können. Eine Ausrichtung des Sensors kann auch anhand eines anderen Anlagenbestandteils erfolgen, mittels dessen die Bearbeitungsoberfläche des trennend bearbeiteten Baumstammes geführt ist.

Es ist auch denkbar, dass der bildgebende Sensor mit seiner Messachse gegenüber der Bearbeitungsoberfläche winklig ausgerichtet ist, insbesondere in einem Winkel zwischen 0 und 90°. Infolge der räumlichen Erstreckung des Tiefenschärfbereichs des bildgebenden Sensors entlang der Messachse kann die Bearbeitungsoberfläche auch bei der oben genannten winkligen Ausrichtung gegenüber der Messachse hinreichend scharf erfasst werden. Insbesondere ist es mittels eines Korrekturschrittes eine trapezförmige Verzerrung der erfassten Bearbeitungsoberfläche auszugleichen, beispielsweise mittels einer Keystone-Korrektur.

In Verfahrensschritt D) erfolgt, wie oben beschrieben, eine ortsaufgelöste Bestimmung des Holzdefektes und/oder der Holzdefekteigenschaft. Die Erfindung ist grundsätzlich nicht darauf beschränkt, welche Art von Holzdefekt oder Holzdefekteigenschaft ermittelt wird. Es ist allerdings relevant, dass diese ortsaufgelöst in Bezug auf die Geometrie des trennend bearbeiteten Baumstammes ermittelt werden können. In anderen Worten wird für den Holzdefekt oder Holzdefekteigenschaft an der Bearbeitungsoberfläche eine räumliche Position und/oder Erstreckung an dem trennend bearbeiteten Baumstamm ermittelt. Insbesondere erfolgt Verfahrensschritt D) unabhängig von einer geometrischen Information an der Bearbeitungsoberfläche des trennend bearbeiteten Baumstammes.

Ein Holzdefekt kann einen Ast und/oder ein Astloch und/oder ein Riss und/oder eine Verwerfung und/oder eine Verfärbung und/oder Fäulnis und/oder Splintholz und/oder Insektenbefall umfassen. Eine Holzdefekteigenschaft kann eine Ausprägung eines Holzdefektes, insbesondere eines der voranstehenden Holzdefekte, beschreiben.

Die Ermittlung des Holzdefektes kann einen Datenverarbeitungsschritt umfassen, mittels dessen das bei der bildgebenden Prüfung erhobene Prüfbild ausgewertet wird. Bevorzugt kann dies eine Bildverarbeitung umfassen, bei der insbesondere eine Filterung und/oder Kantenerkennung und/oder Segmentierung und/oder morphologische Operation und/oder eine Merkmalsextraktion und/oder eine Mustererkennung und/oder eine Musterklassifizierung und/oder eine Texturanalyse und/oder eine Histogrammanalyse an dem Prüfbild oder eines Teils davon durchgeführt wird, um den Holzdefekt und/oder die Holzdefekteigenschaft zu erfassen. Insbesondere kann die ermittelte Position und/oder Ausbreitung des Holzdefektes an dem Prüfbild mittels eines Fehlerpolygons repräsentiert sein.

In einer vorteilhaften Weiterbildung wird in Verfahrensschritt A) ein virtuelles Stammmodell erzeugt, welches die Stammgeometrie zumindest teilweise repräsentiert und mit zumindest einem Profilschnitt versehen wird. Dabei wird eine virtuelle Bearbeitungsebene am virtuellen Stammmodell erzeugt. Die ortsaufgelöste Bestimmung des Holzdefektes erfolgt, indem das Prüfbild oder eine daraus abgeleitete Information in der virtuellen Bearbeitungsebene auf das virtuelle Stammmodell projiziert wird.

Die vorstehend beschriebene Weiterbildung beruht auf der Erkenntnis, dass der Baumstamm in Verfahrensschritt B) typischerweise derart bearbeitet wird, dass die dabei entstehende Bearbeitungsfläche an dem Stammmodell des Verfahrensschrittes A) virtuell nachgebildet werden kann. In anderen Worten wird anhand des Stammmodells ein virtueller Zwilling des Baumstammes erzeugt, welcher einerseits auf der Profilmessung aus Verfahrensschritt A) beruht und andererseits auf einer Anpassung des Stammmodells, insbesondere entsprechend der trennenden Bearbeitung gemäß Verfahrensschritt B). Anhand dieses virtuellen Zwillings erfolgt die Qualitätsprüfung des Baumstammes, indem das Prüfbild oder ein Teil davon in den geschnittenen Bereich des Stammmodells eingesetzt werden kann. Bildhaft kann dieser virtuelle Zwilling des Baumstammes grundsätzlich einem Zylinder oder Hohlzylinder entsprechen, welcher an einer Seite abgeflacht ist und an der abgeflachten Seite mit dem Prüfbild oder einem Teil davon versehen ist.

Insbesondere kann anstelle des Prüfbildes das Fehlerpolygon, welches die Lage des Holzdefektes in dem Prüfbild angibt, auf das Stammmodell projiziert werden. Ein derartiges Fehlerpolygon kann aus einem vorgelagerten Datenverarbeitungsschritt des Prüfbildes stammen. In anderen Worten ist es nicht erforderlich, das Stammmodell und das Prüfbild oder Teile davon visuell zusammenzuführen, damit der Holzdefekt ortsaufgelöst in Bezug auf die Geometrie des trennend bearbeiteten Baumstamms ermittelt werden kann. Vielmehr kann die Erkennung des Holzdefektes und/oder der Holzdefekteigenschaft getrennt von der Ermittlung der Position und/oder Erstreckung des betreffenden Holzdefektes an dem trennend bearbeiteten Baumstamm erfolgen.

In einer vorteilhaften Weiterbildung wird also in Verfahrensschritt D) der Holzdefekt und/oder die Holzdefekteigenschaft anhand des Prüfbildes erkannt und eine Position und/oder Ausbreitung des Holzdefektes und/oder der Holzdefekteigenschaft gegenüber dem trennend bearbeiteten Baumstamm in Abhängigkeit eines gemeinsamen Merkmals des Prüfbildes und des Stammmodells ermittelt.

Ein Vorteil der vorstehend beschriebenen Weiterbildung besteht darin, dass die Erkennung des Holzdefektes und/oder der Holzdefekteigenschaft in einfacher Weise zunächst in einem ersten Teilschritt D1) ausschließlich anhand des Prüfbildes erfolgen kann. Erst in einem darauffolgenden zweiten Teilschritt D2) kann das gemeinsame Merkmal ermittelt werden, welches sowohl in dem Prüfbild als auch dem Stammmodell enthalten ist. Dadurch kann das Prüfbild oder derjenige Teil, welcher den Holzdefekt enthält, hochgenau in die virtuelle Bearbeitungsebene des Stammmodells projiziert werden und damit ortsaufgelöst ermittelt werden, wie der Holzdefekt und/oder die Holzdefekteigenschaft an dem trennend bearbeiteten Baumstamm angeordnet ist.

In einer vorteilhaften Weiterbildung wird in der virtuellen Bearbeitungsebene durch den Profilschnitt am virtuellen Stammmodell zumindest eine virtuelle Waldkante erzeugt, in deren Abhängigkeit die ortsaufgelöste Bestimmung des Holzdefektes und/oder der Holzdefekteigenschaft erfolgt.

Im Rahmen der Erfindung kann der Begriff Waldkante grundsätzlich als ein geometrisches Merkmal an einem trennend bearbeiteten Baumstamm verstanden werden, welcher die Bearbeitungsoberfläche und einen unbearbeiteten Waldkantenbereich voneinander trennt. Der Waldkantenbereich kann als ein außenumfangsseitiger Bereich des Baumstammes mit einer abgerundeten Geometrie angesehen werden, die durch den natürlichen Wuchs des Baumstammes bedingt ist.

Untersuchungen haben gezeigt, dass zumindest eine Waldkante als Referenzmerkmal dienen kann, um das Prüfbild oder einen Teil davon bei der Projektion auf das virtuelle Stammmodell ausrichten zu können und damit eine hohe Genauigkeit bei der ortsaufgelösten Bestimmung des Holzdefektes und/oder der Holzdefekteigenschaft zu erreichen. Insbesondere können durch den Profilschnitt auch zwei virtuelle Waldkanten erzeugt werden, welche in der virtuellen Bearbeitungsebene eine virtuellen Bearbeitungsoberfläche einschließen und damit einen Projektionsbereich vorgeben, in welchen das Prüfbild oder eine daraus abgeleitete Information, zum Beispiel ein Fehlerpolygon, projiziert werden kann.

In einer vorteilhaften Weiterbildung erfolgt die bildgebende Prüfung in Verfahrensschritt C) derart, dass zumindest eine an die Bearbeitungsoberfläche angrenzende Waldkante erfasst wird und in Verfahrensschritt D) die ortsaufgelöste Bestimmung des Holzdefektes und/oder der Holzdefekt in Abhängigkeit eines Vergleichs zwischen der virtuellen Waldkante des virtuellen Stammmodells und der an die Bearbeitungsoberfläche angrenzenden Waldkante des trennend bearbeiteten Baumstammes erfolgt.

Untersuchungen der Anmelderin haben gezeigt, dass die Waldkante sich im Wesentlichen entlang der Stammachse erstreckt und dabei in einer durch die Bearbeitungsoberfläche gebildeten Ebene einen Profilverlauf aufweisen kann, welcher insbesondere individualisierend für den trennend bearbeiteten Baumstamm ist. Eine virtuelle Waldkante kann analog hierzu einen Verlauf aufweisen, der durch den Profilschnitt des virtuellen Stammmodells in der virtuellen Bearbeitungsebene entsteht. Untersuchungen der Anmelderin haben ferner gezeigt, dass die Verläufe einer tatsächlich erzeugten Waldkante und einer virtuellen Waldkante zumindest in der virtuellen Bearbeitungsebene üblicherweise hinreichend ähnlich sind, sodass es möglich ist, das Prüfbild anhand des Waldkantenverlaufs an dem virtuellen Stammmodell auszurichten.

Es liegt im Rahmen der vorteilhaften Weiterbildung, dass der Vergleich zwischen der virtuellen Waldkante des virtuellen Stammmodells und der an die Bearbeitungsoberfläche angrenzenden Waldkante des trennend bearbeiteten Baumstammes mittels einer Recheneinheit und einer darauf implementierten Auswerteroutine erfolgt. Insbesondere ist es denkbar, dass die jeweiligen Waldkanten mit Methoden der Datenverarbeitung, insbesondere mittels eines analytischen Algorithmus und/oder auf Basis maschinellen Lernens oder einer künstlichen Intelligenz, erkannt werden und ermittelt wird, in welcher Position und/oder Größe und/oder Orientierung das Prüfbild oder daraus abgeleitete Information, insbesondere das Fehlerpolygon, in der virtuellen Bearbeitungsebene positioniert werden muss. Die ortsaufgelöste Bestimmung des Holzdefektes und/oder Holzdefekteigenschaft kann anschließend in Abhängigkeit davon erfolgen.

In einer vorteilhaften Weiterbildung wird für den Profilschnitt eine Lage und/oder Orientierung der virtuellen Bearbeitungsebene an dem virtuellen Stammmodell in Abhängigkeit der nominellen Drehlage des Baumstamms in Verfahrensschritt B) definiert.

Die vorstehend beschriebene Weiterbildung beruht auf der Erkenntnis, dass der Baumstamm für seine Verarbeitung üblicherweise um seine Stammachse gedreht wird, bevor er gegen die jeweils eingesetzten Trennmittel bewegt werden kann, um in Verfahrensschritt B) die Bearbeitungsoberfläche zu erzeugen. Die erforderliche Drehlage wird in Abhängigkeit der Stammgeometrie ermittelt. Insofern ist es anhand der Stammgeometrie in Verfahrensschritt A) und der nominellen Drehlage des Baumstammes in Verfahrensschritt B) auch möglich zu ermitteln, in welcher Position und Orientierung die virtuelle Bearbeitungsebene an dem virtuellen Stammmodell angeordnet sein muss, um dieses mit dem Profilschnitt zu versehen. Die vorteilhafte Weiterbildung beruht auch auf der Erkenntnis, dass eine Drehlage des Baumstammes für die trennende Bearbeitung in Verfahrensschritt B) typischerweise hinreichend genau eingestellt werden kann, sodass die erzeugte Bearbeitungsoberfläche sich in der gewünschten Position und Orientierung an dem Baumstamm befindet. In anderen Worten kann die Lage und/oder Orientierung der virtuellen Bearbeitungsebene anhand der Annahme definiert werden, dass zwischen der nominellen Drehlage und der tatsächlichen Drehlage des Baumstammes bei der trennenden Bearbeitung in Verfahrensschritt B) keine Abweichung vorliegt.

In einer denkbaren Ausführungsform wird der Baumstamm also zwischen Verfahrensschritt A) und der trennenden Bearbeitung in Verfahrensschritt B) um seine Stammachse gedreht, um den Baumstamm mit dem zu entfernenden Schwartenbereich in die erforderliche Position für die trennende Bearbeitung zu bringen. Die Ermittlung der hierfür erforderlichen, nominellen Drehlage des Baumstammes kann in Abhängigkeit der Stammgeometrie aus Verfahrensschritt A) erfolgen und diese in ein entsprechendes Steuerprogramm umgesetzt werden, in dessen Abhängigkeit die Drehung und Vorschubbewegung des Baumstammes erfolgt. Mit dieser Steuerinformationen ist es mittels einer Recheneinheit beispielsweise möglich, die virtuelle Bearbeitungsebene derart zu positionieren, dass diese in Bezug auf die virtuelle Stammgeometrie im Wesentlichen identisch zu der Bearbeitungsfläche des trennend bearbeiten Baumstamms vorliegt und diesen damit hinreichend genau repräsentiert.

Aufgrund der natürlichen Gestalt von Baumstämmen oder anderen Störeinflüssen in der industriellen Verarbeitung von Baumstämmen ist denkbar, dass der Baumstamm bei der trennenden Bearbeitung in Verfahrensschritt B) einen Eindrehfehler aufweist und die Bearbeitungsoberfläche mit einer Abweichung gegenüber ihrer gewünschten Position und/oder Orientierung am Baumstamm erzeugt wird. Sofern das Stammmodell in diesem Fall unter Annahme der nominellen Drehlage des Baumstammes mit dem Profilschnitt versehen wird, ergeben sich Unterschiede zwischen der Bearbeitungsoberfläche und den Waldkanten gegenüber ihren virtuellen Gegenstücken am Stammmodell.

Untersuchungen der Anmelderin haben gezeigt, dass es mittels der bildgebenden Prüfung möglich ist, die Bearbeitungsoberfläche oder etwa den Verlauf einer daran angrenzenden Waldkante anhand des Prüfbildes zu ermitteln und anschließend zu bestimmen, in welcher Lage und/oder Orientierung sich die virtuelle Bearbeitungsebene befinden muss, damit sich durch einen Profilschnitt des Stammmodells eine identische oder zumindest ähnliche virtuelle Bearbeitungsfläche und/oder virtuelle Waldkante ergibt.

In einer vorteilhaften Weiterbildung wird hierfür eine Lage und/oder Orientierung der virtuellen Bearbeitungsebene an dem virtuellen Stammmodell in Abhängigkeit der Bearbeitungsoberfläche und/oder zumindest einer Waldkante des Prüfbildes definiert. Insbesondere wird die virtuelle Bearbeitungsebene an dem virtuellen Stammmodell definiert, indem eine virtuelle Bearbeitungsoberfläche und/oder virtuelle Waldkante des Stammmodells ermittelt wird, welche jeweils zu der Bearbeitungsoberfläche und/oder Waldkante des Prüfbildes identisch oder zumindest ähnlich ist. Die Lage und/oder Orientierung der virtuellen Bearbeitungsebene wird derart gewählt, dass diese durch die ermittelte virtuelle Bearbeitungsoberfläche und/oder virtuelle Waldkante verläuft und diese insbesondere enthält.

Die Ermittlung der virtuellen Bearbeitungsebene kann beispielsweise erfolgen, indem das Stammmodell iterativ in einer Vielzahl von unterschiedlichen Positionen und Winkeln geschnitten wird. In den dabei entstehenden Schnittebenen kann ein Abgleich gegenüber der Bearbeitungsoberfläche und/oder Waldkante des Prüfbildes erfolgen. Insbesondere kann ein Ähnlichkeitskriterium definiert sein, in dessen Abhängigkeit die Lage der virtuellen Bearbeitungsebene festgelegt wird. Es ist auch denkbar, dass die virtuelle Bearbeitungsebene ermittelt wird, indem der die Bearbeitungsoberfläche und/oder der Waldkantenverlauf sowie das Stammmodell in ein Berechnungsmodell eingegeben werden, und die Position und/oder Orientierung der virtuellen Bearbeitungsebene explizit und insbesondere in einem einzigen Berechnungsschritt ermittelt wird.

In einer vorteilhaften Weiterbildung wird in einem Verfahrensschritt E) eine Schnittlösung in Abhängigkeit des ortsaufgelösten Holzdefektes und/oder einer Holzdefekteigenschaft ermittelt oder optimiert.

Eine Schnittlösung kann eine Anordnung von Haupt- und/oder Seitenwarebrettern sowie ihre jeweiligen Abmessungen gegenüber der Stammgeometrie umfassen. Im Rahmen des Verfahrensschrittes E) kann die Schnittlösung neu ermittelt werden oder eine bereits bestehende Schnittlösung optimiert werden, insbesondere im Rahmen einer Reoptimierung. Insbesondere kann eine erste Schnittlösung bereits in Abhängigkeit der Profilmessung in Verfahrensschritt A) ermittelt werden, in deren Abhängigkeit Verfahrensschritt B) durchgeführt wird. Nach der trennenden Bearbeitung und der bildgebenden Prüfung des Verfahrensschrittes C) und des Verfahrensschrittes D) kann die ortsaufgelöste Ermittlung des Verfahrensschrittes D) dazu genutzt werden, eine zweite Schnittlösung zu ermitteln, in welcher der Holzdefekt und/oder die Holzdefekteigenschaft berücksichtigt sind. Hierbei kann ein defektbehafteter Stammbereich eliminiert sein oder die Abmessung und/oder Position eines zu erzeugenden Holzbretts gegenüber der ersten Schnittlösung verändert sein. Insbesondere kann auch eine Unterteilung zweier Seitenwarebretter hinsichtlich ihrer relativen Lage zueinander und ihrer Abmessungen gegenüber der ersten Schnittlösung verändert sein.

In einer vorteilhaften Weiterbildung wird der Baumstamm nach Verfahrensschritt E) entsprechend der Schnittlösung trennend bearbeitet. Dies umfasst insbesondere eine weitergehende trennende Bearbeitung des Baumstamms, bei der beispielsweise die Seitenware am Baumstamm profiliert und anschließend vom Baumstamm getrennt wird.

Wie oben erwähnt, wird die Aufgabe auch gelöst durch eine Vorrichtung nach Anspruch 12. Diese umfasst ein Fördermittel, welches dazu vorgesehen ist, einen Baumstamm entlang seiner Stammachse in einer Vorschubrichtung zu transportieren, einen Profilsensor, welcher derart angeordnet ist, um bei der Förderbewegung des Baumstammes eine Stammgeometrie zu ermitteln. Ferner umfasst die Vorrichtung ein erstes Trennmittel, welches derart angeordnet und dazu ausgebildet ist, um mit dem Baumstamm während der Förderbewegung in einen trennenden Eingriff zu gelangen und eine Bearbeitungsoberfläche zu erzeugen, einen Bildsensor, welcher derart angeordnet ist, um die Bearbeitungsoberfläche zu prüfen, ein Recheneinheit, welche dazu eingerichtet ist, einen Holzdefekt und/oder eine Holzdefekteigenschaft an dem trennend bearbeiteten Baumstamm ortsaufgelöst und in Abhängigkeit des Prüfbildes und der Stammgeometrie zu bestimmen.

Insbesondere ist die erfindungsgemäße Vorrichtung dazu geeignet, das erfindungsgemäße Verfahren oder eine vorteilhafte Weiterbildung davon durchzuführen. Insbesondere ist das erfindungsgemäße Verfahren oder eine vorteilhafte Weiterbildung davon mittels der erfindungsgemäßen Vorrichtung oder einer vorteilhaften Weiterbildung davon durchführbar. Insofern gelten hinsichtlich der denkbaren Ausgestaltungen der Vorrichtung sowie der damit erzielbaren Vorteile die Ausführungen bezüglich des oben beschriebenen Verfahrens entsprechend.

Vorteile und mögliche Ausgestaltungen des erfindungsgemäßen Verfahrens sind nachfolgend anhand von Ausführungsbeispielen und den Figuren erläutert. Es zeigen
- Figur 1: einen Baumstamm, dessen Stammgeometrie ermittelt und daraus ein virtuelles Stammmodel erzeugt wird;
- Figur 2: den Baumstamm bei einer trennenden Bearbeitung und der Anpassung des Stammmodells;
- Figur 3: eine bildgebende Prüfung des Baumstammes und Projektion eines Prüfbildes auf das virtuelle Stammmodell;
- Figur 4: eine Anpassung des Stammmodells in Abhängigkeit eines Prüfbildes;
- Figur 5: eine weitergehende trennende Bearbeitung in Abhängigkeit der bildgebenden Prüfung.

In der industriellen Holzverarbeitung werden Baumstämme üblicherweise zu Brettern verarbeitet, die aus unterschiedlichen Querschnittsbereichen der Baumstämme gewonnen werden. Diejenigen Bretter, die aus einem innenliegenden Querschnittsbereich eines Baumstammes gewonnen werden, werden als Hauptwarebretter bezeichnet. Bretter, die im Vergleich dazu aus einem außenliegenden Querschnittsbereich gewonnen werden, werden als Seitenwarebretter bezeichnet. Seitenwarebretter werden üblicherweise unmittelbar am Baumstamm profiliert. Hierbei wird ein Schwartenbereich am Stück oder durch Verarbeitung zu Hackschnitzeln vom Baumstamm entfernt, wodurch eine im Wesentlichen ebene Bearbeitungsoberfläche erzeugt wird. Die Bearbeitungsoberfläche stellt hierbei meist die Breitseite eines Seitenwarebretts dar. Sofern der Baumstamm an mehreren Seiten bearbeitet wird, kann er nach der trennenden Bearbeitung als sogenanntes Model mit zwei seitlichen Bearbeitungsoberflächen oder etwa als Kantling mit vier umfangsseitig verteilten Bearbeitungsoberflächen vorliegen. Nach der trennenden Bearbeitung wird der trennend bearbeitete Baumstamm mittels zumindest eines Profilsensors gescannt. Dadurch können innenliegende Holzdefekte mit einer dreidimensionalen Erstreckung, wie beispielsweise Risse oder Äste, erkannt werden.

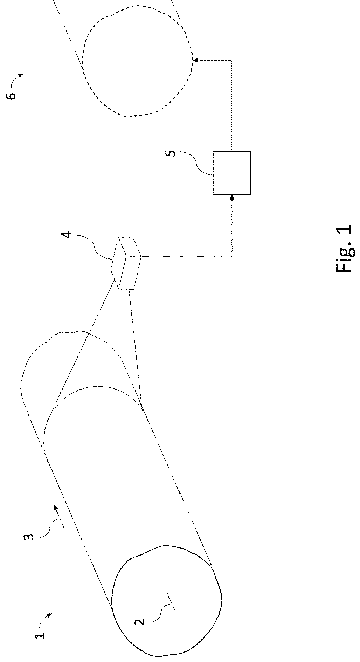

Die oben beschriebenen Maßnahmen der Qualitätsprüfung und Bearbeitung von Baumstämmen mit einem hohen messtechnischen Aufwand einher, ergeben sich jedoch durch die gängige Praxis in der Verfahrensführung bei der Holzbearbeitung. Anhand der nachfolgenden Figuren 1 bis 5 sind Verfahrensschritte beschrieben, die die Erreichung vergleichbarer Vorteile mit einem deutlich geringeren messtechnischen Aufwand ermöglichen.

Figur 1 zeigt einen Baumstamm 1, welcher eine im Wesentlichen zylindrische Grundgeometrie aufweist, die sich entlang seiner Stammachse 2 erstreckt. Der Baumstamm 1 weist ein unregelmäßiges Oberflächenprofil auf, welches durch seinen natürlichen Wuchs bedingt ist.

Der Baumstamm 1 wird in einer Vorschubrichtung 3 gefördert und gelangt dabei in einen Erfassungsbereich mehrerer Profilsensoren 4, von denen in dem hier gezeigten Ausführungsbeispiel nur ein Profilsensor 4 gezeigt ist. Die Profilsensoren 4 können beispielhaft jeweils als Laser-Lichtschnittsensoren ausgebildet sein. Eine Stammgeometrie des Baumstammes 1 wird in Abhängigkeit der Vorschubbewegung, insbesondere einer Relativgeschwindigkeit gegenüber den Profilsensoren 4, ermittelt, indem mehrere Profilbilder in eine Recheneinheit 5 gegeben werden, die anhand einer Punktewolke ein virtuelles Stammmodell 6 erzeugt. Das virtuelle Stammmodell 6 weist zumindest außenumfangsseitig eine Geometrie auf, die im Wesentlichen der Geometrie des Baumstammes 1 entspricht.

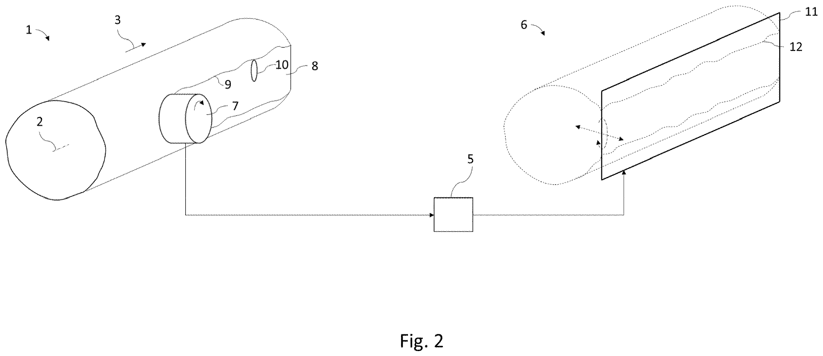

In einem darauffolgenden Schritt, welcher in Figur 2 dargestellt ist, wird der Baumstamm 1 mittels eines rotierenden Messerkopfes 7 bearbeitet, wodurch eine im Wesentlichen ebene Bearbeitungsoberfläche 8 erzeugt wird, die seitlich durch zwei Waldkanten 9 begrenzt ist. Zur besseren Übersicht ist in Figur 2 nur eine der Waldkanten 9 mit einem Bezugszeichen versehen. In hier ebenfalls nicht näher gezeigter Weise kann der trennend bearbeitete Baumstamm 1 nicht nur eine, sondern beispielsweise zwei oder etwa vier umfangsseitig verteilte Bearbeitungsoberflächen 8 aufweisen und nach der trennenden Bearbeitung als sogenanntes Model bzw. Kantling vorliegen.

Die Waldkanten 9 erstrecken sich im Wesentlichen entlang der Stammachse 2 und weisen zumindest in einer Ebene, die durch die Bearbeitungsoberfläche 8 gebildet ist, einen charakteristischen Verlauf auf, welcher durch den natürlichen Wuchs des Baumstammes 1 bedingt ist. Infolge der trennenden Bearbeitung des Baumstammes 1 wird an der Bearbeitungsoberfläche 8 ein Holzdefekt 10 freigelegt. Dieser wirkt sich, wie einführend erwähnt, negativ auf die Qualität der zu gewinnenden Bretter aus, deren Oberfläche zumindest teilweise durch die Bearbeitungsoberfläche 8 begrenzt sind.

Um den Holzdefekt 10 für die weitere Bearbeitung des Baumstammes 1 berücksichtigen zu können, wird das virtuelle Stammmodell 6 in einer virtuellen Bearbeitungsebene 11 mit einem Profilschnitt versehen. Entlang dieses Profilschnittes erstrecken sich zwei virtuelle Waldkanten 12, von denen in Figur 2 nur eine mit Bezugszeichen versehen ist.

Die Lage und Orientierung der virtuellen Bearbeitungsebene 12 am virtuellen Baumstamm 6 wird in Abhängigkeit der nominellen Drehlage des Baumstammes 1 erzeugt, nachdem dieser trennend bearbeitet wurde. Diese liegt üblicherweise in der Recheneinheit 5 vor, die die trennende Bearbeitung des Baumstammes 1 sowie seine Förderbewegung steuert. Mit diesen Steuerinformationen ist es möglich, nominelle Drehlage des Baumstammes 1 bei der trennenden Bearbeitung zu ermitteln und die virtuelle Bearbeitungsebene 11 derart gegenüber dem virtuellen Stammmodell 6 zu positionieren, dass sie entsprechend der Ebene verläuft, in der die tatsächlich erzeugte Bearbeitungsoberfläche 8 an dem Baumstamm 1 liegt.

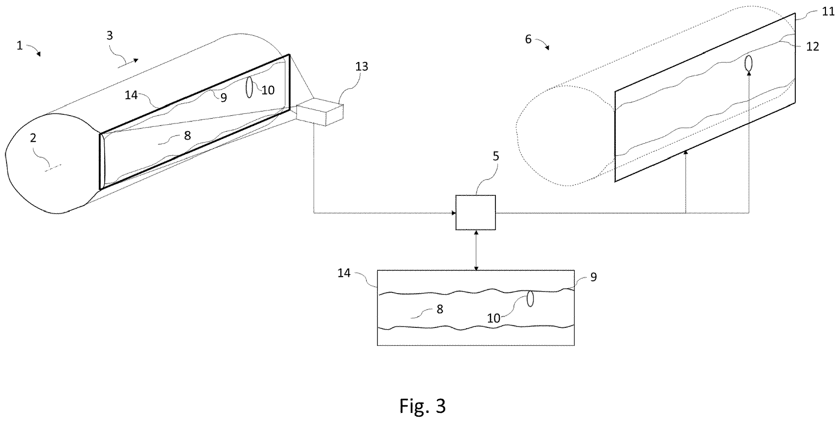

Nach den in Figur 2 veranschaulichten Schritten, wird der Baumstamm 1 weiter in Vorschubrichtung 3 gefördert und gelangt, wie in Figur 3 gezeigt, in einen Erfassungsbereich mindestens eines bildgebenden Sensors 13. Dieser ist derart orientiert, dass die Bearbeitungsoberfläche 8 im Wesentlichen orthogonal zu einer Messachse des bildgebenden Sensors 13 angeordnet ist. Eine derartige Orientierung des Baumstamms 1 kann beispielsweise durch geeignete Ansteuerung der Förder- oder Handhabungsmittel erfolgen, mittels derer der Baumstamm in Vorschubrichtung 3 bewegt wird.

Mittels des bildgebenden Sensors 13 wird ein Prüfbild 14 erfasst, welches sowohl die Bearbeitungsoberfläche 8, der darin liegende Holzdefekt 10, als auch die Waldkanten 9 des trennend bearbeiteten Baumstammes 1 vollständig erfasst. In hier nicht gezeigter Weise kann auch jeweils nur ein Teil der Bearbeitungsoberfläche 8 und/oder der Waldkanten 9 erfasst werden.

Anhand des Prüfbildes 14 wird mittels der Recheneinheit 5 zunächst der Holzdefekt 10 erkannt. Dies kann mittels digitaler Bildverarbeitung erfolgen oder etwa mittels eines Algorithmus auf Basis maschinellen Lernens oder künstlicher Intelligenz. Anschließend wird das Prüfbild 14 oder nur der erkannte Holzdefekt 10 oder ein dazu korrespondierendes Fehlerpolygon in die virtuelle Bearbeitungsebene 11 des virtuellen Stammmodells 6 projiziert. Es ist relevant, dass das Prüfbild 14 hierfür anhand der darin enthaltenen Waldkanten 9 mit den virtuellen Waldkanten 12 abgeglichen wird und das Prüfbild 14 oder Holzdefekt 10 oder Fehlerpolygon in Abhängigkeit davon an dem virtuellen Stammmodell 6 positioniert wird. Dadurch ist es möglich, die Position und Erstreckung des Holzdefektes 12 und seine Holzdefekteigenschaften hochgenau und ortsaufgelöst in Bezug auf die Stammgeometrie des Baumstammes 1 zu bestimmen.

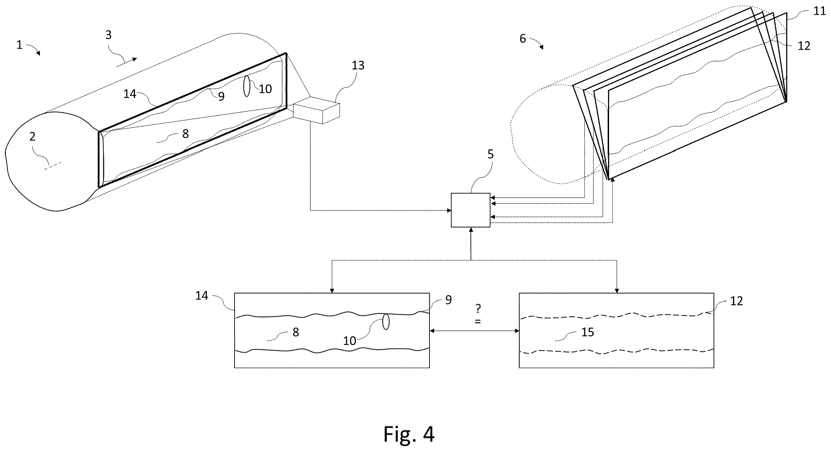

Aufgrund von unvermeidbaren Ungenauigkeiten bei der Positionierung kann der Baumstamm 1 bei der trennenden Bearbeitung einen Eindrehfehler aufweisen und die Bearbeitungsoberfläche mit einer Abweichung gegenüber ihrer gewünschten Position oder Orientierung am Baumstamm 1 erzeugt werden. Wie anhand von Figur 4 gezeigt ist, ist es mittels der bildgebenden Prüfung der Bearbeitungsoberfläche 8 am trennend bearbeiteten Baumstamm 1 möglich, die Gestalt oder Abmessung der Bearbeitungsoberfläche 8 oder etwa den Verlauf einer oder zweier daran angrenzenden Waldkanten 9 anhand des Prüfbildes 14 zu ermitteln. Anschließend kann an dem Stammmodell 6 bestimmt werden, in welcher Lage und/oder Orientierung sich die virtuelle Bearbeitungsebene 11 befinden muss, damit sich durch einen Profilschnitt des Stammmodells 6 eine identische oder zumindest ähnliche virtuelle Bearbeitungsfläche 15 oder virtuelle Waldkante 12 ergibt.

Wie anhand von Figur 4 beispielhaft gezeigt ist, kann das Stammmodell 6 zum Beispiel iterativ mit einer Mehrzahl an möglichen Schnittebenen geschnitten werden und anhand des jeweiligen Schnittes entsprechend viele virtuelle Bearbeitungsoberflächen 15 oder virtuelle Waldkanten 12 am Stammmodell 6 ermittelt werden. Die Recheneinheit 5 vergleicht das erfasste Prüfbild 14 und insbesondere die darin enthaltene Bearbeitungsoberfläche 8 und/oder Waldkante 9 mit den Schnitten des Stammmodells 6 und den darin enthaltenen virtuellen Bearbeitungsoberflächen 15 und virtuellen Waldkanten 12. Sofern eine hinreichende Ähnlichkeit vorliegt, wird die betreffende Schnittebene als virtuelle Bearbeitungsebene 11 definiert und in diese das Prüfbild 14 oder etwa ein Defektpolygon projiziert. Es ist auch denkbar, dass die Position und/oder Orientierung der virtuellen Bearbeitungsebene 11 ermittelt wird, indem der die Bearbeitungsoberfläche und/oder der Waldkantenverlauf sowie das Stammmodell 6 in ein Berechnungsmodell eingegeben werden, und die Position und/oder Orientierung der virtuellen Bearbeitungsebene 11 anhand eines Ähnlichkeitskriteriums explizit und insbesondere in einem Berechnungsschritt ermittelt wird.

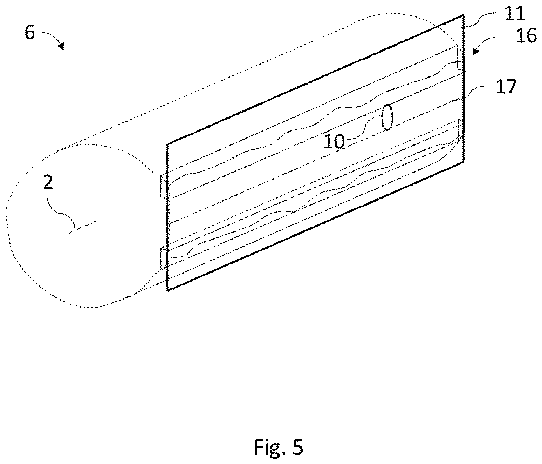

Durch eine genaue ortsaufgelöste Ermittlung des Holzdefektes 10 oder der Holzdefekteigenschaften ist es möglich, eine Schnittlösung zu ermitteln, entsprechend derer der Baumstamm 1 für eine optimale Holzausbeute bearbeitet werden kann. Dies ist anhand von Figur 5 veranschaulicht. Demnach kann etwa die Seitenware durch Ausfräsen zweier daran angrenzender Waldkantenbereiche 16 profiliert werden oder etwa die Unterteilung zweier benachbarter Seitenwarebretter durch eine Ritzebene 17 verändert werden, sodass nur eines der Seitenwarebretter von dem Holzdefekt 10 betroffen ist und das jeweils andere Seitenwarebrett nicht.

## Patentansprüche

1. Verfahren zur Erfassung von Holzdefekten und/oder Holzdefekteigenschaften bei der Verarbeitung eines Baumstamms mit den folgenden Verfahrensschritten:
A) Profilmessung des Baumstammes (1) und Ermittlung einer Stammgeometrie des Baumstamms;
B) Trennende Bearbeitung des Baumstammes (1) unter Erzeugung einer Bearbeitungsoberfläche (8) entlang einer Stammachse des Baumstamms (1);
C) Bildgebende Prüfung des trennend bearbeiteten Baumstammes (1) zumindest an der Bearbeitungsoberfläche (8) und Erfassung mindestens eines Prüfbildes (14);
D) Ortsaufgelöste Bestimmung eines Holzdefektes (10) und/oder einer Holzdefekteigenschaft an dem trennend bearbeiteten Baumstamm in Abhängigkeit des Prüfbildes (14) aus Verfahrensschritt C) und der Stammgeometrie aus Verfahrensschritt A).

2. Verfahren nach Anspruch 1, wobei
anhand der Stammgeometrie aus Verfahrensschritt A) ein virtuelles Stammmodell (6) erzeugt wird und das virtuelle Stammmodell (6) in einer virtuellen Bearbeitungsebene (11) mit zumindest einem Profilschnitt versehen wird und wobei die ortsaufgelöste Bestimmung des Holzdefektes und/oder der Holzdefekteigenschaft erfolgt, indem das in Verfahrensschritt C) ermittelte Prüfbild (14) oder eine daraus abgeleitete Information, in der virtuellen Bearbeitungsebene auf das virtuelle Stammmodell (6) projiziert wird.

3. Verfahren nach Anspruch 2, wobei
Verfahrensschritt D) zwei Teilschritte D1) und D2) umfasst, wobei
in Teilschritt D1) der Holzdefekt (10) und/oder die Holzdefekteigenschaft in dem Prüfbild (14) erkannt wird und
in Teilschritt D2) eine Position und/oder Ausbreitung des Holzdefektes und/oder der Holzdefekteigenschaft an dem trennend bearbeiteten Baumstamm (1) in Abhängigkeit eines gemeinsamen Merkmals zwischen dem Prüfbild (14) und dem virtuellen Stammmodell (16) ermittelt wird.

4. Verfahren zumindest nach Anspruch 2, wobei
durch den Profilschnitt in der virtuellen Bearbeitungsebene (11) zumindest eine virtuelle Waldkante (12), insbesondere zwei virtuelle Waldkanten (12), am Stammmodell (6) erzeugt wird, in deren Abhängigkeit die ortsaufgelöste Bestimmung des Holzdefektes (10) und/oder der Holzdefekteigenschaft erfolgt.

5. Verfahren nach Anspruch 4, wobei
in Verfahrensschritt B) die bildgebende Prüfung derart erfolgt, dass eine an die Bearbeitungsoberfläche (8) angrenzende Waldkante (9) erfasst wird und in Verfahrensschritt D) die ortsaufgelöste Bestimmung des Holzdefektes und/oder der Holzdefekteigenschaft in Abhängigkeit eines Vergleichs zwischen der virtuellen Waldkante (12) des Stammmodells (6) und der an die Bearbeitungsoberfläche (8) angrenzende Waldkante (9) des trennend bearbeiteten Baumstammes (1) erfolgt.

6. Verfahren zumindest nach Anspruch 2, wobei
eine Lage und/oder Orientierung der virtuellen Bearbeitungsebene (11) an dem virtuellen Stammmodell (6) in Abhängigkeit einer nominellen Drehlage des Baumstamms (1) in Verfahrensschritt B) definiert wird.

7. Verfahren zumindest nach Anspruch 2, wobei
eine Lage und/oder Orientierung der virtuellen Bearbeitungsebene (11) an dem virtuellen Stammmodell (6) in Abhängigkeit der Bearbeitungsoberfläche (8) und/oder zumindest einer Waldkante (9) des Prüfbildes (14) definiert wird.

8. Verfahren nach Anspruch 7, wobei
die virtuelle Bearbeitungsebene (11) an dem virtuellen Stammmodell (6) definiert wird, indem eine virtuelle Bearbeitungsoberfläche (15) und/oder virtuelle Waldkante (12) ermittelt wird, welche zu der Bearbeitungsoberfläche (8) und/oder Waldkante (9) des Prüfbildes (14) identisch oder zumindest ähnlich ist, und die Lage und/oder Orientierung der virtuellen Bearbeitungsebene (11) insbesondere derart gewählt wird, dass diese die ermittelte virtuelle Bearbeitungsoberfläche (15) und/oder die virtuelle Waldkante (12) enthält.

9. Verfahren nach Anspruch 7, wobei
Verfahrensschritt D) unabhängig von einer geometrischen Information an der Bearbeitungsoberfläche (8) des trennend bearbeiteten Baumstammes (1) erfolgt.

10. Verfahren nach einem der voranstehenden Ansprüche, wobei
in einem Verfahrensschritt E) eine Schnittlösung in Abhängigkeit des ortsaufgelösten Holzdefektes (10) und/oder der Holzdefekteigenschaft ermittelt wird.

11. Verfahren nach Anspruch 10, wobei
der Baumstamm (1) nach Verfahrensschritt E) entsprechend der Schnittlösung trennend bearbeitet wird.

12. Vorrichtung zur Verarbeitung eines Baumstamms, insbesondere mittels eines Verfahrens nach einem der Ansprüche 1 bis 11, mit
einem Fördermittel, welches dazu vorgesehen ist, einen Baumstamm (1) entlang seiner Stammachse in einer Vorschubrichtung (3) zu transportieren,
einem Profilsensor (4), welcher derart angeordnet ist, um bei der Förderbewegung des Baumstammes (1) eine Stammgeometrie zu ermitteln,
einem ersten Trennmittel (7), welches derart angeordnet und dazu ausgebildet ist, um mit dem Baumstamm (1) während der Förderbewegung in einen trennenden Eingriff zu gelangen und eine Bearbeitungsoberfläche zu erzeugen
einem Bildsensor (13), welcher derart angeordnet ist, um die Bearbeitungsoberfläche (8) zu prüfen und ein Prüfbild (14) auszugeben,
einer Recheneinheit (5), welche dazu eingerichtet ist, einen Holzdefekt (10) und/oder eine Holzdefekteigenschaft an dem trennend bearbeiteten Baumstamm ortsaufgelöst und in Abhängigkeit des Prüfbildes (14) und der Stammgeometrie zu bestimmen.
